# EUROPEAN PATENT APPLICATION

(11) **EP 4 825 840 A1**
(43) Date of publication of application: **21.10.2026**
(21) Application number: 24902979.4
(22) Date of filing: 13.12.2024
(51) Int. Cl.: C07D 207/404, C08G 65/333, C08G 65/332, C08G 73/10

(54) **POLYESTER SYNTHESIS BASED ON ASPARTIC ACID MODIFICATION**

(30) Priority: 15.12.2023 CN 202311738254
(71) Applicant: Mint Biotechnologies Co., Ltd., Hangzhou, Zhejiang 310012 (CN)
(72) Inventor: ZHANG, Kechun, Hangzhou, Zhejiang 310012 (CN); CHEN, Xi, Hangzhou, Zhejiang 310012 (CN); LEI, Tengfei, Hangzhou, Zhejiang 310012 (CN); LIU, Shengyang, Hangzhou, Zhejiang 310012 (CN)
(74) Representative: Gille Hrabal Partnerschaftsgesellschaft mbB Patentanwälte
(86) International application number: PCT/CN2024/139269
(87) International publication number: WO 2025/124564

(57) **Abstract**

Provided in the present invention is polyester synthesis based on aspartic acid modification. A monomer of a unique structure of an imide ring is obtained based on aspartic acid and a dibasic acid likely to be cyclized, the monomer is based on the aspartic acid, can be derived from biomass, is low in cost and renewable, meets the requirement of sustainable development, and the unique structure of the monomer can regulate and control various properties of polyester materials, such as the mechanics, temperature resistance, hydrophilicity and hydrophobicity, and crystallization.

## Description

### TECHNICAL FIELD

The present disclosure belongs to the technical field of polymer synthesis, and particularly relates to polyester synthesis based on aspartic acid modification.

### BACKGROUND

Currently, resin materials on the market still face the challenge of achieving a balance between costs and performance, and this is especially true for bio-based materials. Polybutylene succinate (PBS), as degradable plastic, offers good heat resistance and finds wide application, but it suffers from poor mechanical strength and high costs. Polylactic acid (PLA), which has good degradability, exhibits relatively good mechanical strength, but the material is brittle, has poor resistance to both high and low temperatures, and lacks toughness. Polyethylene terephthalate (PET), currently a major focus of research on recyclable materials, is widely applied to food packaging, yet its heat resistance is also unsatisfactory. Although the recently popular polyethylene furandicarboxylate (PEF) material shows improved heat resistance, its currently high monomer cost limits large-scale application.

Research on high-performance bio-based materials with significant cost advantages and green monomer sources represents a viable path to addressing the poor environmental friendliness, high recycling costs, and poor performance of current purely petroleum-based resin materials.

### SUMMARY

The present disclosure first provides a diacid monomer containing an imide ring structure represented by Formula I, or an ester or prepolymer thereof: 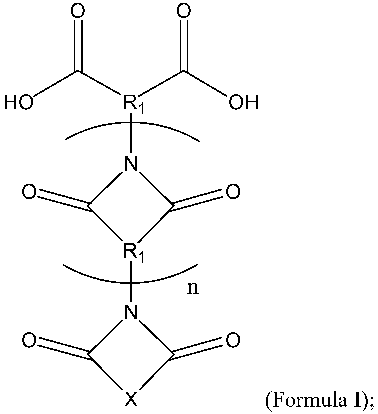
wherein R₁ is a derived group of aspartic acid, and X is a residue of a readily cyclizable diacid or -C=C-;
n is 0 or an integer greater than 0; and preferably, n is any integer from 0 to 7;
or, the monomer includes structures obtained after ring opening of the diacid monomer containing the imide ring structure represented by Formula I or the ester or prepolymer thereof, for example: 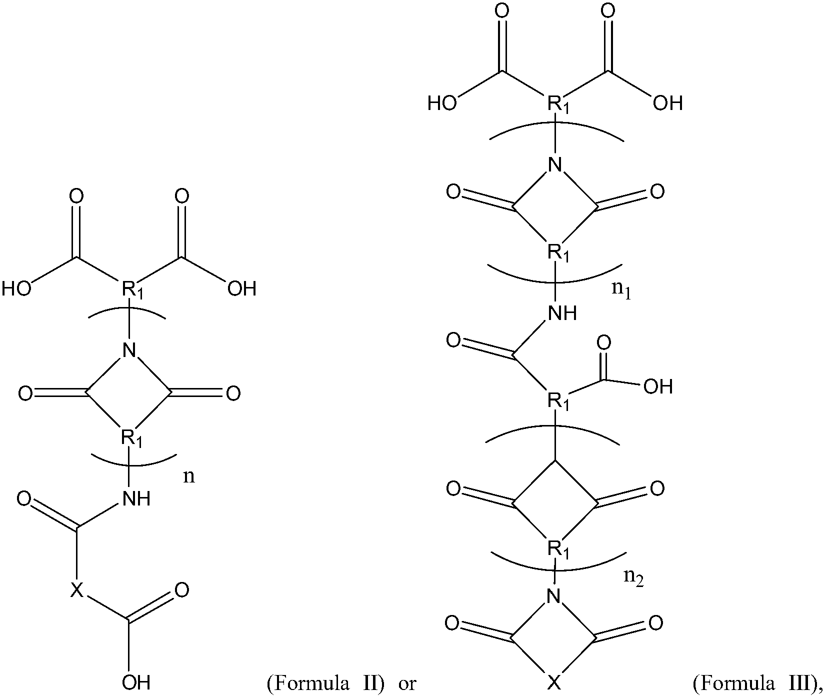 or a combination of ring opening manners of (Formula II) and (Formula III), wherein n₁ and n₂ are each a non-negative integer less than n, and preferably n₁ and n₂ are each any integer from 0 to 6;
an N value is independently selected from n, n₁ or n₂; and preferably the content of monomers with the N value greater than 0 is greater than 0;
X is preferably -C=C-; or X is preferably at least one of alkylene unsubstituted or substituted by a substituent selected from halogen, alkyl, aryl, arylalkyl or alkylaryl, alkylene unsubstituted or substituted by a substituent selected from halogen, alkyl, aryl, arylalkyl or alkylaryl and interrupted by one or more O atoms, alkenylene unsubstituted or substituted by a substituent selected from halogen, alkyl or nitro, cycloalkylene or heterocycloalkylene unsubstituted or substituted by a substituent selected from halogen, alkyl or nitro, cycloalkenylene unsubstituted or substituted by a substituent selected from halogen, alkyl or nitro, arylene unsubstituted or substituted by a substituent selected from halogen, alkyl or nitro, heteroarylene unsubstituted or substituted by a substituent selected from halogen, alkyl or nitro, or a bridged cyclic group unsubstituted or substituted by a substituent selected from halogen, alkyl or nitro;
preferably, the alkylene in definition of X is C1-C10 alkylene, preferably C1-C6 alkylene, more preferably C1-C5 alkylene, and most preferably methylene, ethylene, 1-methylethylene, 1,1-dimethylethylene, 1,2-dimethylethylene, 1,1,2,2-tetramethylethylene, 1-phenylethylene, 1-phenylmethylethylene, 1,1-diphenylethylene, 1,1-diphenylmethylethylene, 1,2-diphenylethylene, 1,2-diphenylmethylethylene, propylene, 1-methylpropylene, 2-methylpropylene, 1,1-dimethylpropylene, 1,2-dimethylpropylene, 2,2-dimethylpropylene, 1,3-dimethylpropylene, 1-phenylpropylene, 2-phenylpropylene, 1,2-diphenylpropylene, 2,2-diphenylpropylene, or 1,3-diphenylpropylene; the alkenylene in the definition of X is C2-C10 alkenylene, preferably C2-C6 alkenylene, more preferably C2-C3 alkenylene, and most preferably vinylene, 1-methylvinylene, 1,2-dimethylvinylene, or propenylene; the alkylene unsubstituted or substituted by the substituent selected from halogen, alkyl, aryl, arylalkyl or alkylaryl and interrupted by one or more O atoms in the definition of X is interrupted by 1 to 3 oxygen atoms, such as 2-oxa-1,3-propylene; the cycloalkylene in the definition of X is C3-C8 cycloalkylene, preferably C4-C6 cycloalkylene, and most preferably cyclobutylene or cyclohexylene; the cycloalkenylene in the definition of X is C4-C8 cycloalkenylene, preferably C4-C6 cycloalkenylene, and most preferably 3-cyclohexene-1,6-diyl; the arylene in the definition of X is C6-C10 arylene, preferably phenylene, such as 1,6-phenylene; and bridged cycloalkylene in the definition of X is norbomenylene; and
most preferably, X is -C=C-, ethylene, 1,2-cyclohexylene or 1,2-phenylene.

Preferably, the aspartic acid in the monomer is of biological origin; and preferably, the aspartic acid has a ¹⁴C/¹²C ratio greater than 0.

In a second aspect, the present disclosure provides a monomer composition, comprising the diacid monomer containing the imide ring structure represented by Formula I, or further comprising the diacid monomer represented by Formula II and/or Formula III, or the ester or prepolymer thereof according to claim 1, and comprising two or more monomers with different N values, wherein the N value is independently selected from n, n₁ or n₂, and the content of monomers with the N value greater than 0 is greater than 0.

Preferably, the monomer composition is the diacid monomer containing the imide ring structure represented by Formula I or represented by a mixture of Formula I and Formula II or the ester or prepolymer of the monomer, more preferably a mixture of monomers comprising at least one N value of n being 0 or n being 1 to 7, or more preferably a mixture of monomers comprising at least two N values of n being 1 to 7.

Or, the monomer composition is the diacid monomer containing the imide ring structure represented by a mixture of Formula I and Formula III or a mixture of Formula I, Formula II and Formula III or the ester or prepolymer of the monomer, more preferably a mixture of monomers comprising at least one N value of n being 0 to 7 and monomers comprising at least one N value of n₁ and n₂ being 0 to 6, and most preferably a monomer containing at least one N value of n, n₁ and n₂ being greater than 0.

A third aspect of the present disclosure provides a copolymer, including a polymer backbone structural unit and a first modified structural unit, wherein the first modified structural unit contains a structure shown in Formula VI:

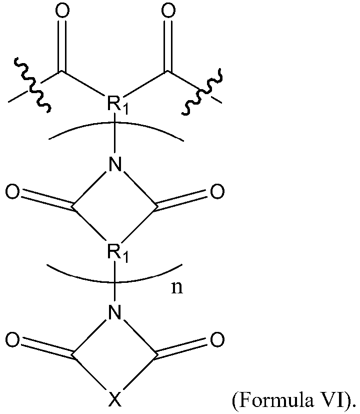

Preferably, the first modified structural unit is a polyester structure shown in (Formula VI-1):

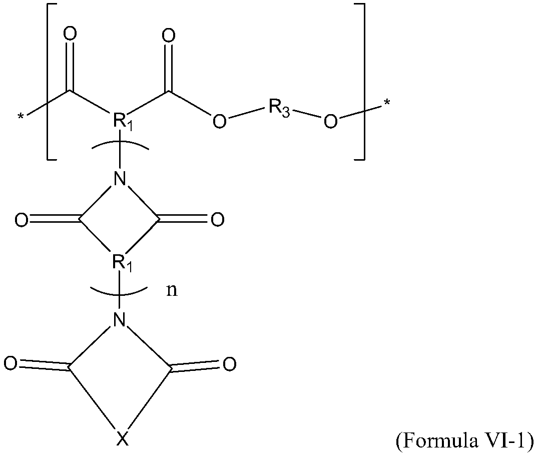

R₃ is a residue of a diol for polymer synthesis, and preferably is selected from C1-C12 alkylene unsubstituted or substituted by a substituent selected from halogen, alkyl, aryl, arylalkyl or alkylaryl, cycloalkylene, or heterocyclylene comprising at least one N, O or S atom; and preferably, R₃ is selected from at least one of C2-C8 alkylene unsubstituted or substituted by a substituent selected from halogen, alkyl, aryl, arylalkyl or alkylaryl, C4-C8 cycloalkylene or C5-C8 heterocyclylene comprising an oxygen atom, and is further preferably C2-C6 alkylene.

Preferably, the first modified structural unit further contains a structure shown in Formula VII and/or Formula VIII: 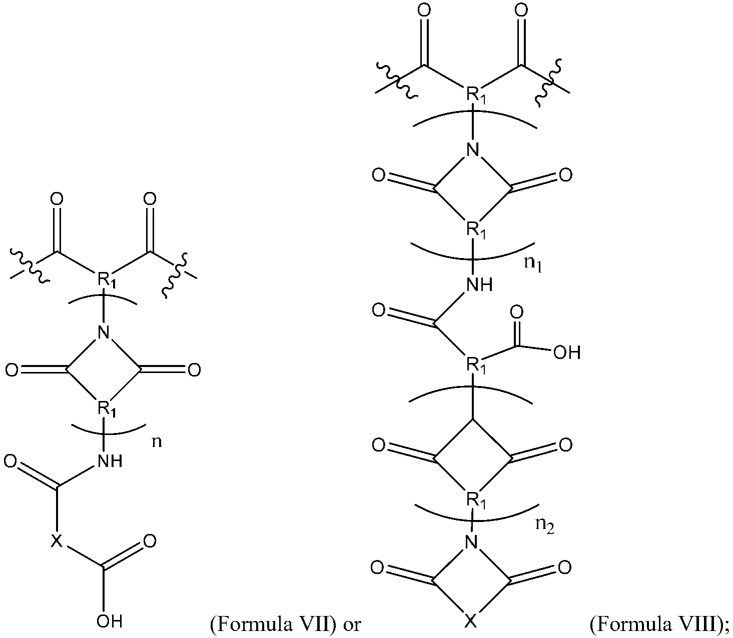
n is 0 or an integer greater than 0, and preferably n is any integer from 0 to 7; n₁ and n₂ are each a non-negative integer less than n, and preferably n₁ and n₂ are each any integer from 0 to 6; and preferably, an N value is independently selected from n, n₁ or n₂, and the content of monomers with the N value greater than 0 is greater than 0;
preferably, the first modified structural unit further contains a polyester structure shown in (Formula VII-1) and/or (Formula VIII-1): 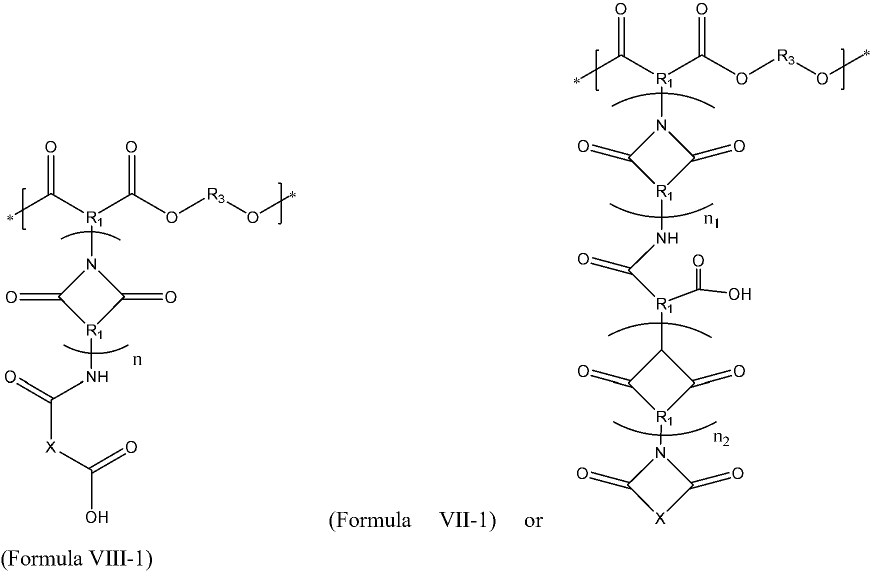
and preferably, the first modified structural unit forms a branched and/or cross-linked structure through double bonds and/or a carboxyl group on a side chain.

In a specific implementation of the present disclosure, the polymer backbone structural unit is polyester; preferably, the backbone structural unit is polyester such as one or more of aliphatic polyester, aliphatic-aromatic polyester, and aromatic polyester; further preferably, the backbone structural unit contains two or more different structural units; and
preferably, the backbone structural unit is a structural unit of one or more polyesters of PBS, PES, PBA, PET, PBT, PTT, PBAT, PBST, PEAT or PEST.

In a specific implementation of the present disclosure, the first modified structural unit accounts for 0.5-99.5mol% of total polymer structural units, preferably 1-90 mol%, and further preferably 5-30 mol%.

In a specific implementation of the present disclosure, the copolymer further contains a second modified structural unit.

The present disclosure further provides a polymer alloy including the above copolymer.

The present disclosure further provides a polymer composition or molded article including the above copolymer or the above polymer alloy.

The present disclosure further provides use of the above copolymer, the above polymer alloy or the above composition or molded article in food containers, food packaging films, disposable tableware such as spoons or straws, daily necessities, cosmetics, transparent boxes for household appliances and the like, packaging containers such as transparent windows of paper boxes, stationery such as transparent folders and card holders, industrial films or agricultural films, and chemical fibers for clothing or industrial use.

The present disclosure further provides use of any one of the aforementioned monomer or the ester or prepolymer thereof, or any one of the aforementioned monomer composition, or a precursor composition of the monomer or the monomer composition for polymer synthesis, wherein the precursor composition includes aspartic acid and a readily cyclizable diacid .

Preferably, the monomer or the monomer composition includes at least two, three, four, five or six monomers with an N value of 0, 1, 2, 3, 4 or 5.

Preferably, the monomer or the monomer composition includes monomers with an N value of 0, 1 and 2; and preferably, the monomers with the N value of 0 have the highest content.

Preferably, the monomer or the monomer composition includes monomers with an N value of 1, 2, 3, 4 and 5; and preferably, the monomers with the N value of 2 have the highest content.

Preferably, the monomer or the monomer composition includes monomers with an N value of 2, 3, 4 and 5; and preferably, the monomers with the N value of 3 have the highest content.

The present disclosure further provides a method for synthesizing the above monomer or monomer composition, including a step of reacting aspartic acid with a readily cyclizable diacid or an anhydride thereof, wherein preferably, a molar ratio of the aspartic acid to the readily cyclizable diacid is 1:50-50:1, preferably 1:10-10:1, and further preferably 1:3-1:1.

Preferably, the reaction is carried out in a molten condition.

The present disclosure further provides a method for synthesizing the above copolymer, including:
providing the above monomer or monomer composition;
providing a polyester prepolymer of a diacid and a diol; and
subjecting the monomer or the monomer composition in Step 1), the prepolymer in Step 2) and optionally a newly added diacid and/or diol to a polycondensation reaction.

### Beneficial Technical Effects

The modifying monomer used in the present disclosure is based on the aspartic acid, can be derived from biomass, is low in cost and renewable, and meets the requirement of sustainable development. Besides, the monomer of the unique structure in the present disclosure can regulate and control various properties of polyester materials, such as mechanical properties, temperature resistance, hydrophilicity and hydrophobicity, and crystallization.

### BRIEF DESCRIPTION OF DRAWING(S)

FIG. 1 is an NMR spectrum of a diacid monomer containing an imide ring structure.
FIG. 2 is analysis of monomer self-polymerization by an NMR spectrum (aspartic acid : butanedioic acid = 1:3).
FIG. 3 is analysis of monomer self-polymerization by an NMR spectrum (aspartic acid : butanedioic acid = 1:5).
FIG. 4 is an NMR spectrum of a product obtained in Monomer Synthesis Example 3 of the present disclosure.
FIG. 5 is an NMR spectrum of a product obtained in Monomer Synthesis Example 4 of the present disclosure.
FIG. 6 is an XRD pattern of PBS modified with a 10% aspartic acid modifying unit.
FIG. 7 is an FTIR spectrum of PBS modified with a 10% aspartic acid modifying unit.
FIG. 8 is a mass spectrum of a product of aspartic acid : butanedioic anhydride = 1: 1.
FIG. 9 is a mass spectrum of a product of aspartic acid : butanedioic anhydride = 1:3.
FIG. 10 is a mass spectrum of a product of aspartic acid : butanedioic anhydride = 1: 10.

### DETAILED DESCRIPTION

In the following description, numerous specific details are set forth to provide a more thorough understanding of the present disclosure. However, it is apparent to those skilled in the art that the present disclosure may be practiced without one or more of these details. In other instances, certain technical features well known in the art have not been described in order to avoid obscuring the present disclosure.

The practice of the present disclosure is described in detail below in combination with terminology definitions.

### I. Aspartic acid monomer containing imide ring structure and synthesis thereof

The present disclosure first provides an aspartic acid monomer containing an imide ring structure, and the monomer has the following structure: 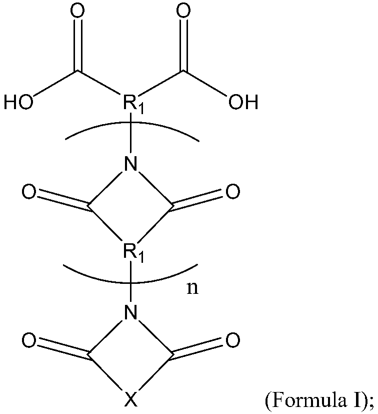 wherein R₁ is a derived group of aspartic acid, X is a residue of a readily cyclizable diacid or - C=C-, and n is 0 or an integer greater than 0.

The derived group of the aspartic acid in the present disclosure is a residual group remaining after a reaction of the aspartic acid with the readily cyclizable diacid, excluding the two carboxyl groups and amino, namely -CH(-)-CH₂-.

The present disclosure has found that, when the readily cyclizable diacid is used to block amino of the aspartic acid, the aspartic acid itself may undergo self-polymerization or ring opening, or form a terminal double bond, resulting in a unique structure having, on a side chain, one or more succinimide rings or ring-opened structures, as well as a readily cyclizable diacid -terminated or aspartic acid terminal double-bond side chain, and the structure has an influence on properties of a polymer.

Therefore, the monomer of the present disclosure may include one or more ring-opened structures of imide (Formula I). For example: 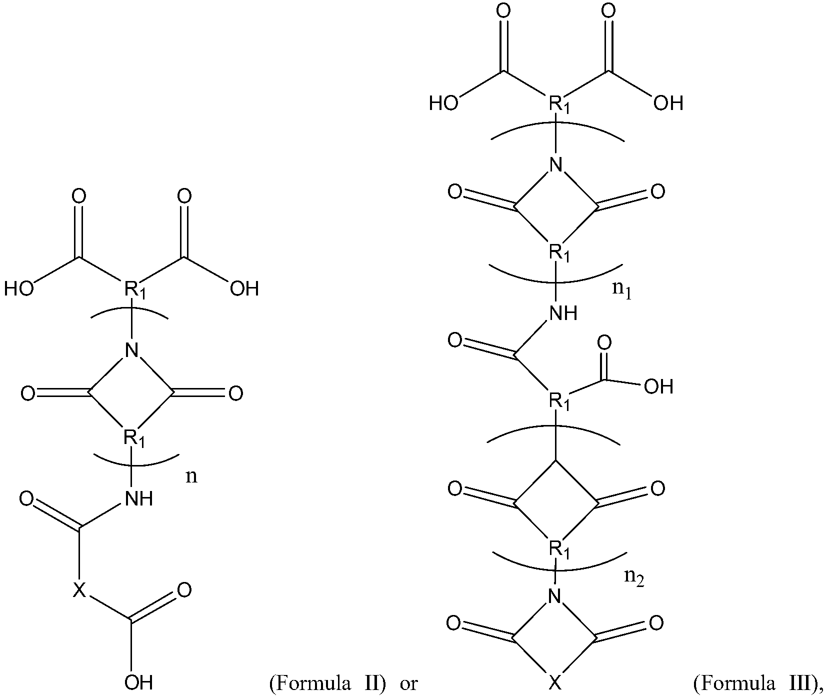 or a combination of the ring-opened structures of (Formula II) and (Formula III).

For the structure of (Formula III), when the amino and two carboxyls in the aspartic acid are interrupted by different carbon atoms, two different ring-opened structures exist: 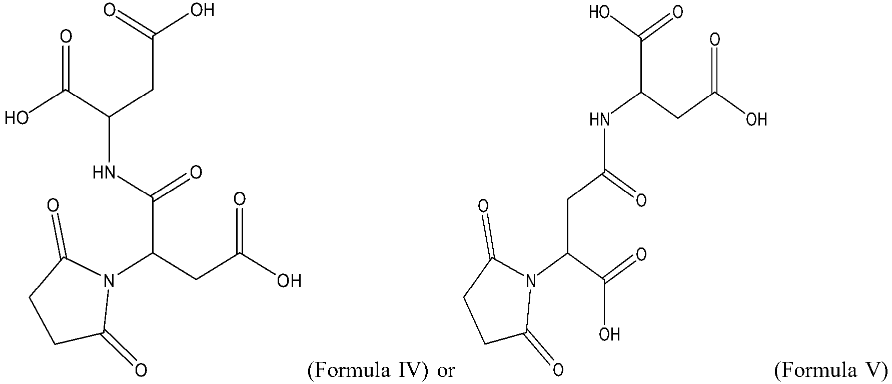 wherein, as exemplified by (Formula IV), the ring opening form in which the carboxyl closer to the amino forms an amide bond is referred to as an α ring-opened structure in the present disclosure; and as exemplified by (Formula V), the ring opening form in which the carboxyl farther from the amino forms an amide bond is referred to as a β ring-opened structure in the present disclosure.

An exemplary aspartic acid monomer containing an imide ring structure includes:

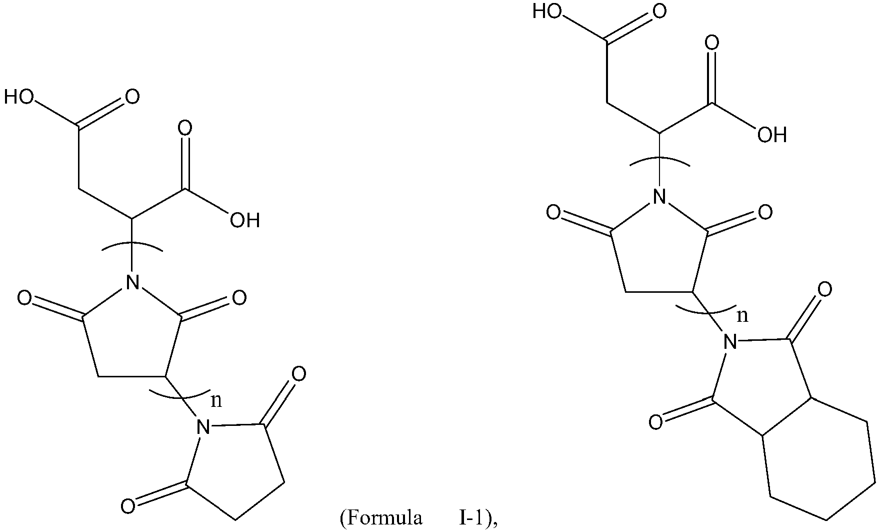

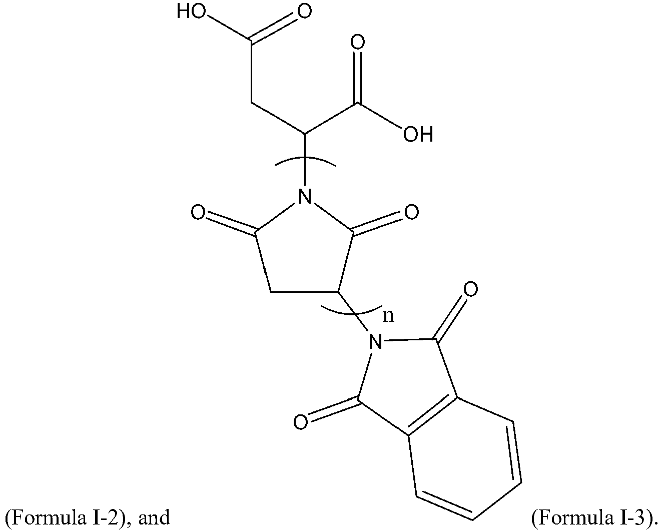

Exemplarily, a typical monomer containing at least one aspartic acid deamination double bond in the present disclosure has a following structure:

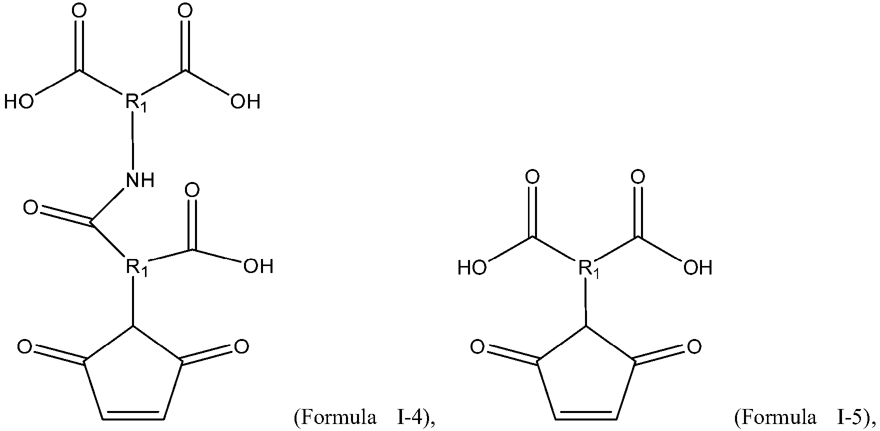

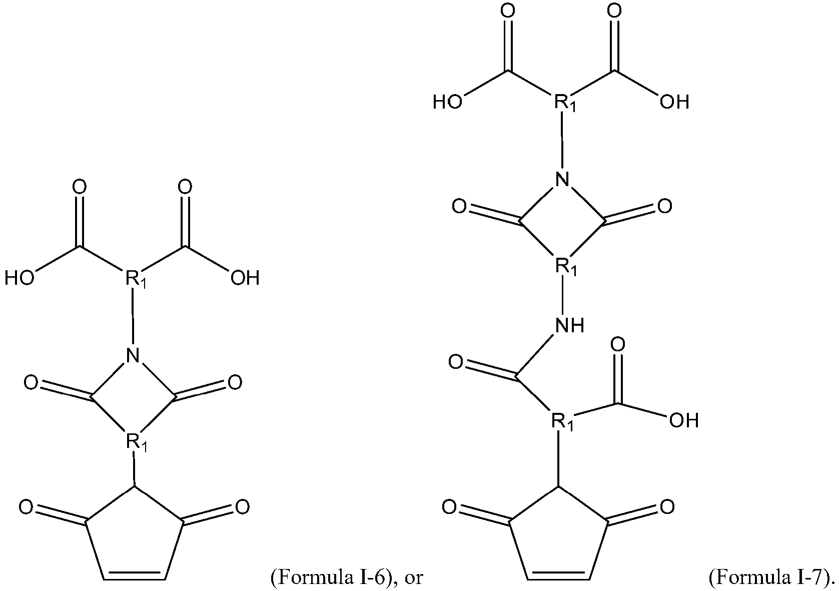

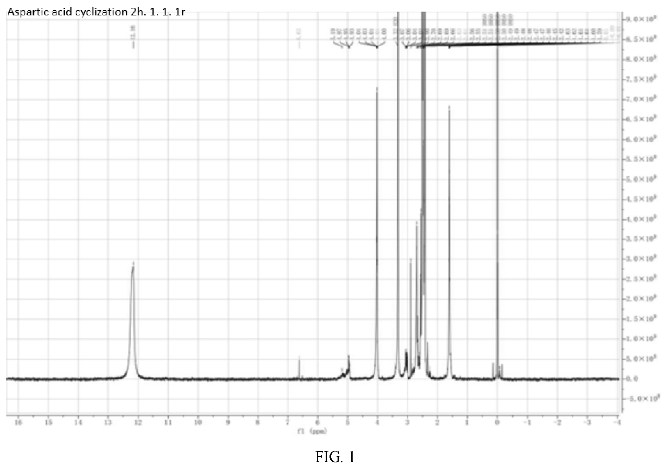

Synthesis of the aspartic acid monomer containing the imide ring structure:
The above monomer can be obtained by mixing the aspartic acid and the readily cyclizable diacid with a certain ratio for an amidation reaction. Reaction conditions can be selected by those skilled in the art according to the aspartic acid and the readily cyclizable diacid, for example, the reaction is carried out under anaerobic melt conditions. See FIG. 1 for an NMR spectrum of a typical monomer. The absence of H absorption peaks of amino and imino at around 8 indicates that the amino of the aspartic acid is completely protected.

An N value is independently selected from n, n₁ or n₂, that is, the value of n, n₁ or n₂ may be the same or different. Formula I, Formula II or Formula III may each contain a plurality of monomers with different N values.

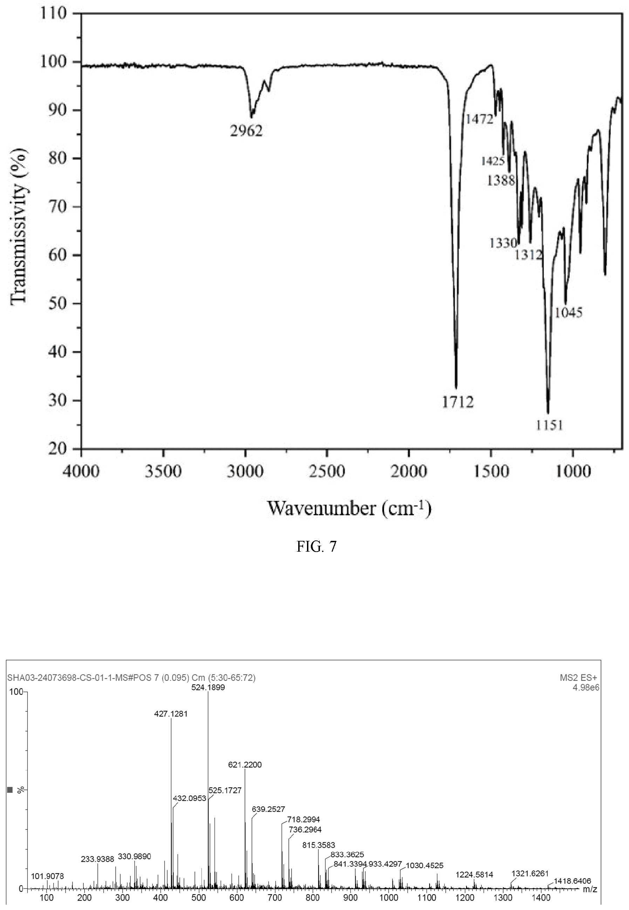

See FIG. 8 to FIG. 10 for electrospray mass spectra of typical monomers or monomer compositions with the N value greater than 0. It is known via analysis that monomers or monomer compositions with different N values can be obtained by changing a feeding ratio of the aspartic acid to the readily cyclizable diacid .

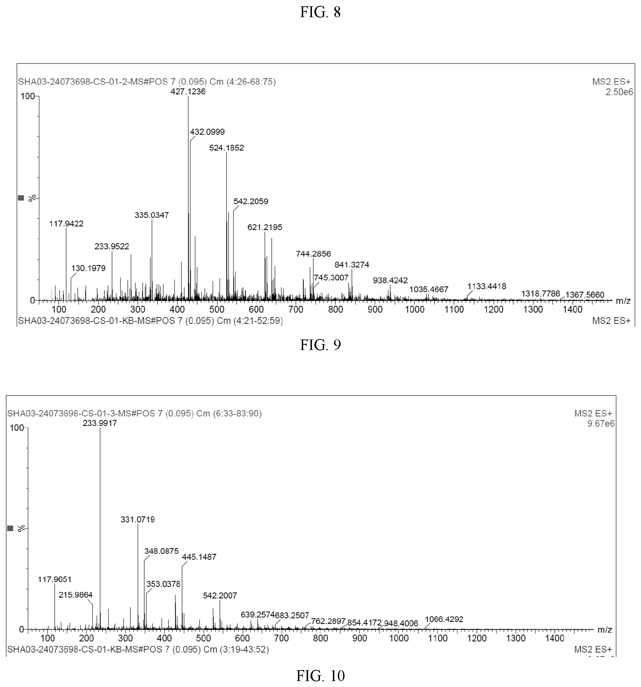

Taking a reaction of aspartic acid with butanedioic anhydride at 180°C for 2 h as an example, when aspartic acid : butanedioic anhydride = 1:1, N=2, 3, 4 and 5 are predominant, among which N=3 has the highest content, followed by N=2 (represented by FIG. 8); when aspartic acid : butanedioic anhydride = 1:3, N=1, 2, 3, 4 and 5 are predominant, among which N=2 has the highest content, followed by N=3 (represented by FIG. 9); and when aspartic acid : butanedioic anhydride = 1:10, N=0, 1 and 2 are predominant, among which N=0 has the highest content, followed by N=1 (represented by FIG. 10). The above analysis of the N value is obtained by integral analysis of spectral curves.

In the practice of polymer synthesis, the diacid may be converted into an ester with a lower alcohol (e.g., C1-C6 alkyl alcohol) or into a prepolymer with a diol, and polymer synthesis may then be carried out. Therefore, esters or prepolymers of the diacid monomer shown in Formula I, or a reaction mixture of the diacid monomer shown in Formula I with a lower alcohol and/or a diol, also falls within the scope of protection of the present disclosure.

### II. First modified structural unit

By introducing the above aspartic acid monomer containing the imide ring structure into a polymer, a first modified structural unit is formed. In specific aspects of the present disclosure, the first modified structural unit is polyester: 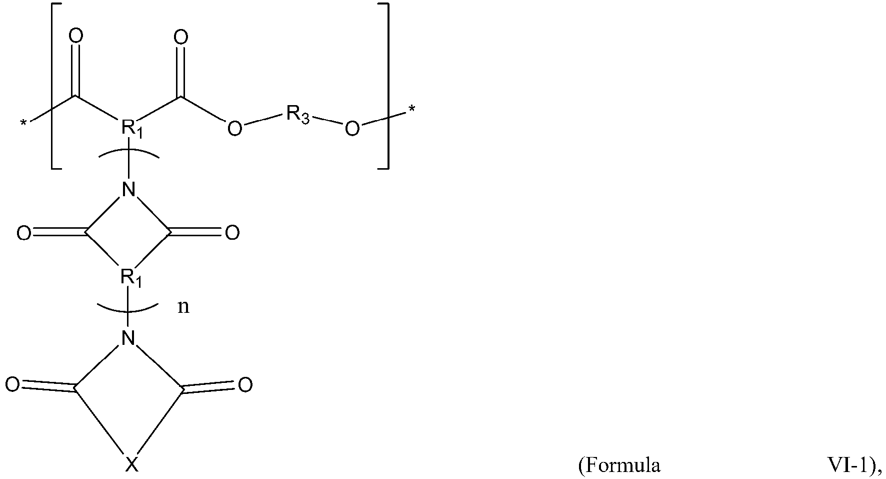 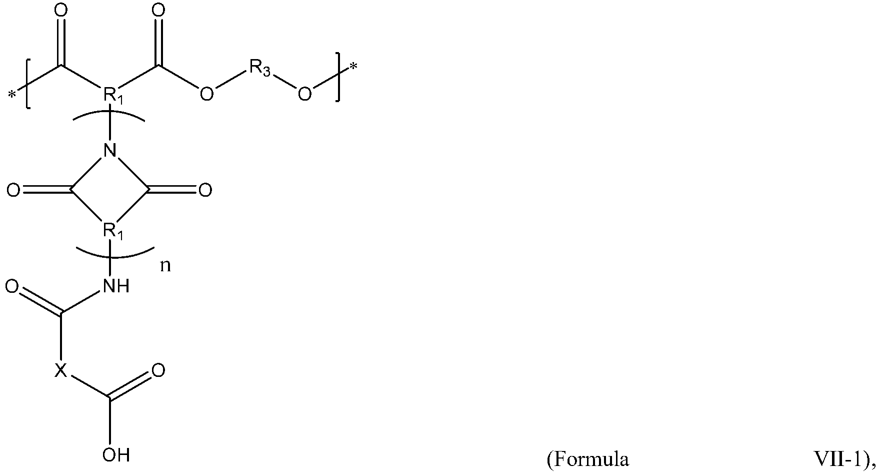 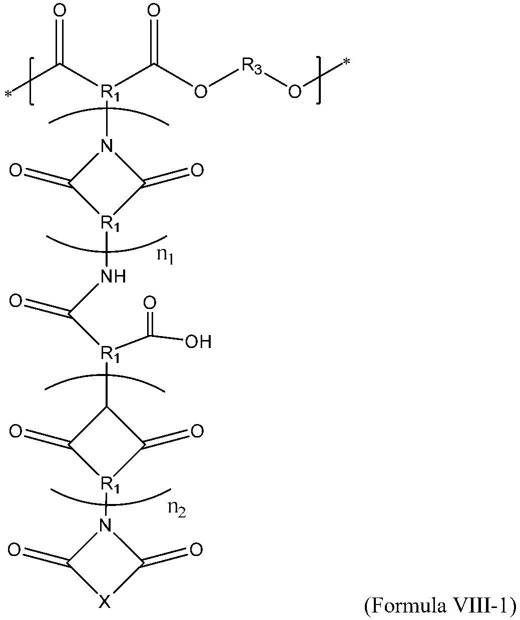 or a combination thereof.

R₃ is a residue of a diol for polymer synthesis.

When the aspartic acid monomer containing the imide ring structure has a ring-opened structure (carboxyl) and/or a double-bond structure, the first modified structural unit also contains the same ring-opened structure (carboxyl) and/or double-bond structure, and branching and/or cross-linking are/is formed in the polymer.

In a further implementation of the present disclosure, a main-chain structural unit of the modified polymer is also polyester: 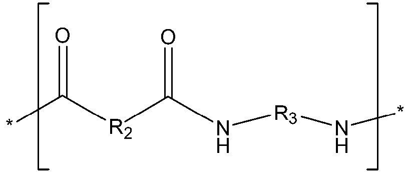 wherein R₂ is a residue of a diacid for polymer synthesis, R₃ is the residue of the diol for polymer synthesis, and R₃ in the main-chain structural unit and R₃ in the first modified structural unit are the same or different.

In a further implementation of the present disclosure, branching and/or cross-linking are/is formed in the polymer through ring-opened carboxyl and/or a double bond on a side chain of the first modifying monomer.

In certain aspects of the present disclosure, the aspartic acid of the first modified structural unit is derived from biomass, is low in cost and renewable, and meets the requirement of sustainable development.

In another aspect of the present disclosure, the first modified structural unit improves the rigidity of the polymer, and in another aspect, improves the temperature resistance of the polymer.

### III. Polymer comprising second modified structural unit

The polymer of the present disclosure may optionally include a second modified unit that has heat resistance, transparency or high barrier properties. The second modified unit may be composed of a polymeric repeating unit formed from a diacid, a diamine, or an amino acid (one or more of isophthalic acid, furandicarboxylic acid, camphoric acid, adipic acid, proline, and m-xylylenediamine) and a diol (one or more of ethylene glycol, butanediol, and 1,4-cyclohexanedimethanol).

### IV. Alloy

The polymers of the present disclosure may form alloys or optionally form alloys with other polymers.

### V. Composition and molded article

The present disclosure further provides a composition or a molded article of the above polymer. Methods for processing or molding various types of polymers are known in the art.

Taking a polyester copolymer as an example, the polyester composition in the present disclosure further contains a plasticizer, a nucleating agent, or a hydrolysis inhibitor, in addition to the strength-improving polymers described above.

In the polyester composition of the present disclosure, as other components besides those mentioned above, a filler (inorganic filler, or organic filler), a flame retardant, an antioxidant, a hydrocarbon wax or an anionic surfactant, i.e., a lubricant, an ultraviolet absorber, an antistatic agent, an anti-corona agent, a light stabilizer, a pigment, an antifungal agent, an antibacterial agent, a foaming agent, and the like may be contained within a range that does not impair the effects of the present disclosure. In addition, other high polymer materials and other resin compositions may also be added within a range that does not impair the effects of the present disclosure.

The polyester composition of the present disclosure may be used to prepare the molded article such as a sheet by extrusion molding or compression molding; and further, the resulting sheet may be subjected to thermoforming, for example, stretching into a film or fiber, within a temperature range that is higher than a glass transition temperature (Tg) and lower than the melting point (Tm) of the polyester resin composition.

### VI. Products and use

The polymer, its alloys or its compositions or molded articles in the present disclosure are suitable for being used in food containers, food packaging films, disposable tableware such as spoons or straws, daily necessities, cosmetics, transparent boxes for household appliances and the like, packaging containers such as transparent windows of paper boxes, stationery such as transparent folders and card holders, industrial films or agricultural films, chemical fibers for clothing or industrial use, etc.

### VII. Technical terminology

### Structural unit and segment

A structural unit, also referred to as a monomer unit, is the smallest non-divisible structural unit included in a polymer. It has the same structure as a monomer except for functional groups involved in polymerization. A binary copolymer contains two types of different structural units and may be classified as a random copolymer, an alternating copolymer, a block copolymer, or the like. The polymer of the present disclosure is preferably a random copolymer.

### Melt flow index

The melt flow index in the present disclosure adopts a test standard ASTM-D1238, is expressed in units of g/10 min, and has a tolerance range of ±5.

### Readily cyclizable dicarboxylic acid HOOC-X-COOH

HOOC-X-COOH is a readily cyclizable diacid, namely a dicarboxylic acid which may form a cyclic anhydride easily without or with a catalyst. The readily cyclizable diacid is known by those skilled in the art, for example, refer to CN110790906B. HOOC-X-COOH may be selected from alkanedicarboxylic acid unsubstituted or substituted by a substituent selected from halogen, alkyl, aryl, arylalkyl or alkylaryl, alkanedicarboxylic acid unsubstituted or substituted by a substituent selected from halogen, alkyl, aryl, arylalkyl or alkylaryl and interrupted by one or more O atoms, alkenedicarboxylic acid unsubstituted or substituted by a substituent selected from halogen or alkyl, cycloalkanedicarboxylic acid unsubstituted or substituted by a substituent selected from halogen, alkyl or nitro, cycloalkenedicarboxylic acid unsubstituted or substituted by a substituent selected from halogen, alkyl or nitro, aromatic dicarboxylic acid unsubstituted or substituted by a substituent selected from halogen, alkyl or nitro, or bridged dicarboxylic acid unsubstituted or substituted by a substituent selected from halogen, alkyl or nitro. Preferably, HOOC-X-COOH may be selected from at least one of butanedioic acid, 2-methylbutanedioic acid, 2-phenylbutanedioic acid, 2-phenylmethylbutanedioic acid, 2,2-dimethylbutanedioic acid, 2,3-dimethylbutanedioic acid, 2,3-diphenylbutanedioic acid, 1,2-cyclobutanedicarboxylic acid, 2,2,3,3-tetramethylbutanedioic acid, methylmaleic acid, dimethylmaleic acid, phthalic acid, hexahydrophthalic acid, norbornenedioic acid, tetrahydrophthalic acid, pentanedioic acid, 2-methylpentanedioic acid, 3-methylpentanedioic acid, 3-phenylpentanedioic acid, 2,2-dimethylpentanedioic acid, 3,3-dimethylpentanedioic acid, diglycolic acid, 2,3-furandicarboxylic acid, 3,4-furandicarboxylic acid, 2,3-pyridinedicarboxylic acid, or 3,4-pyridinedicarboxylic acid. The cyclic anhydride of HOOC-X-COOH may preferably be selected from at least one of butanedioic anhydride, 2-methylbutanedioic anhydride, 2-phenylbutanedioic anhydride, 2-phenylmethylbutanedioic anhydride, 2,2-dimethylbutanedioic anhydride, 2,3-dimethylbutanedioic anhydride, 2,3-diphenylbutanedioic anhydride, 1,2-cyclobutanedicarboxylic anhydride, 2,2,3,3-tetramethylbutanedioic anhydride, methylmaleic anhydride, dimethylmaleic anhydride, phthalic anhydride, hexahydrophthalic anhydride, norbornenedioic anhydride, tetrahydrophthalic anhydride, pentanedioic anhydride, 2-methylpentanedioic anhydride, 3-methylpentanedioic anhydride, 3-phenylpentanedioic anhydride, 2,2-dimethylpentanedioic anhydride, 3,3-dimethylpentanedioic anhydride, diglycolic anhydride, 2,3-furandicarboxylic anhydride, 3,4-furandicarboxylic anhydride, 2,3-pyridinedicarboxylic anhydride, or 3,4-pyridinedicarboxylic anhydride.

### Dicarboxylic acid for polymer synthesis

The dicarboxylic acid for polymer synthesis in the present disclosure may be used for the synthesis of the polymer backbone structural unit, and may also be used for the synthesis of the structural unit for improving polymer strength. When used for the synthesis of the structural unit for improving polymer strength, it is defined as HOOC-R₂-COOH in the present disclosure, which may be any diacid different from HOOC-X-COOH. For example, it may be the readily cyclizable diacid described above for HOOC-X-COOH; or it may be a non-readily cyclizable diacid, such as terephthalic acid, 2,5-furandicarboxylic acid, oxalic acid, propanedioic acid, 1,6-hexanedioic acid, 1,10-decanedioic acid, or 1,18-octadecanedioic acid. Preferably, HOOC-R₂-COOH may be selected from alkanedicarboxylic acid unsubstituted or substituted by a substituent selected from halogen, alkyl, aryl, arylalkyl or alkylaryl, alkanedicarboxylic acid unsubstituted or substituted by a substituent selected from halogen, alkyl, aryl, arylalkyl or alkylaryl and interrupted by one or more O atoms, alkenedicarboxylic acid unsubstituted or substituted by a substituent selected from halogen or alkyl, cycloalkanedicarboxylic acid unsubstituted or substituted by a substituent selected from halogen, alkyl or nitro, cycloalkenedicarboxylic acid unsubstituted or substituted by a substituent selected from halogen, alkyl or nitro, aromatic dicarboxylic acid unsubstituted or substituted by a substituent selected from halogen, alkyl or nitro, or bridged dicarboxylic acid unsubstituted or substituted by a substituent selected from halogen, alkyl or nitro. Preferably, HOOC-R₂-COOH may be selected from at least one of butanedioic acid, 2-methylbutanedioic acid, 2-phenylbutanedioic acid, 2-phenylmethylbutanedioic acid, 2,2-dimethylbutanedioic acid, 2,3-dimethylbutanedioic acid, 2,3-diphenylbutanedioic acid, 1,2-cyclobutanedicarboxylic acid, 2,2,3,3-tetramethylbutanedioic acid, oxalic acid, propanedioic acid, 1,6-hexanedioic acid, 1,10-decanedioic acid, 1,18-octadecanedioic acid, maleic acid, methylmaleic acid, dimethylmaleic acid, phthalic acid, hexahydrophthalic acid, norbornenedioic acid, tetrahydrophthalic acid, pentanedioic acid, 2-methylpentanedioic acid, 3-methylpentanedioic acid, 3-phenylpentanedioic acid, 2,2-dimethylpentanedioic acid, 3,3-dimethylpentanedioic acid, diglycolic acid, 2,3-furandicarboxylic acid, 3,4-furandicarboxylic acid, 2,3-pyridinedicarboxylic acid, 3,4-pyridinedicarboxylic acid, terephthalic acid, or 2,5-furandicarboxylic acid.

### Diol HO-R₃-OH for polymer synthesis

The diol for polymer synthesis may be selected from alkylene glycol unsubstituted or substituted by a substituent selected from halogen, alkyl or nitro, OH-alkylene-cycloalkylene-alkylene-OH unsubstituted or substituted by a substituent selected from halogen, alkyl or nitro, polyether diol, or alkylene glycol interrupted by one or more N atoms; and is preferably selected from at least one of alkylene glycol having 2 to 18 carbon atoms, polyethylene glycol, polypropylene glycol, polytetrahydrofuran diol, N-methyldiethanolamine, or N-ethyldiethanolamine. It may preferably be selected from at least one of ethylene glycol, 1,3-propanediol, 1,4-butanediol, 1,5-pentanediol, 1,6-hexanediol, 1,7-heptanediol, 1,8-octanediol, 1,9-nonanediol, 1,10-decanediol, 1,18-octadecanediol, polyethylene glycol, or 1,4-cyclohexanedimethanol.

### Polyester structural unit

The polyester structural unit used in the present disclosure has the meaning commonly understood in the art, and is preferably formed by polycondensation of the above-mentioned dicarboxylic acid for polymer synthesis and the above-mentioned diol for polymer synthesis.

The present disclosure is further illustrated below with reference to examples. Instances of the examples described are intended to explain the present disclosure and should not be construed as limiting the present disclosure.

### Examples

### Monomer Synthesis Example 1

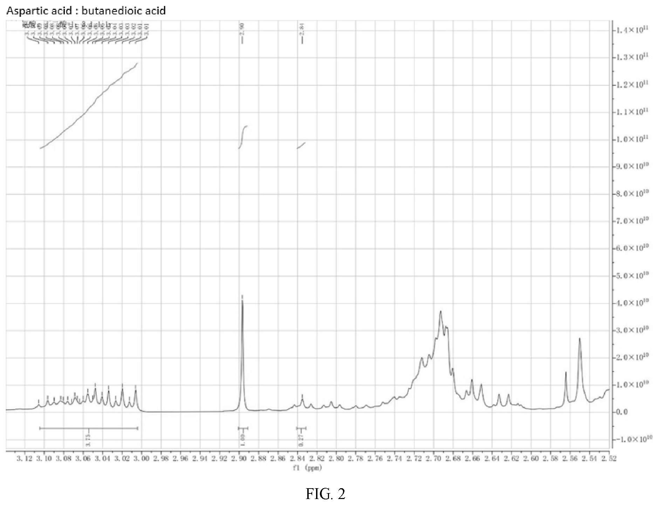

Aspartic acid and butanedioic acid reacted in a reactor according to a molar ratio of 1:3, 200 ppm each of an antioxidant and a thermal stabilizer were added, and a resulting mixture was heated to melt under nitrogen protection so as to react. An NMR spectrum of a resulting product is shown in FIG. 2.

### Monomer Synthesis Example 2

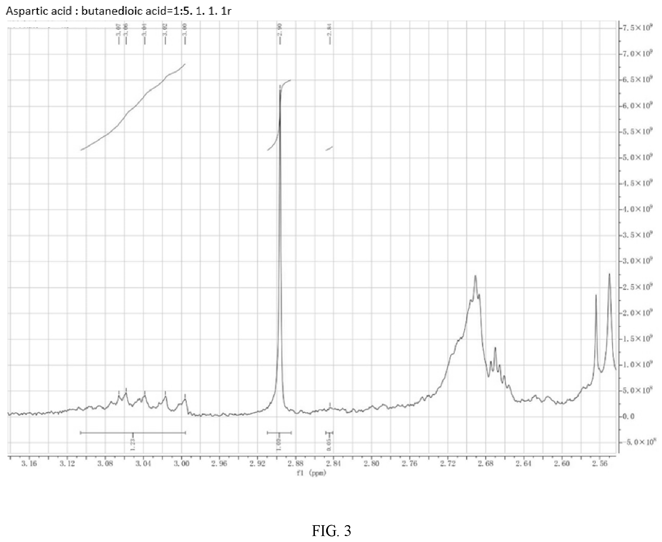

Aspartic acid and butanedioic acid reacted in a reactor according to a molar ratio of 1:5, 200 ppm each of an antioxidant and a thermal stabilizer were added, and a resulting mixture was heated to melt under nitrogen protection so as to react. An NMR spectrum of a resulting product is shown in FIG. 3.

As can be seen from FIG. 2 and FIG. 3, a peak at 2.9 is a methylene absorption peak when the aspartic acid has not undergone self-polymerization, and peaks around 3.08 and 2.84 are methylene absorption peaks on a ring when the aspartic acid self-polymerizes. A ratio is determined by integration, and as the proportion of the aspartic acid increases, i.e., the number of self-polymerized rings increases, it can be seen that the products obtained in Monomer Synthesis Examples 1 and 2 are mixtures of compounds of Formula I-1 with different n values, i.e., the proportion of compounds of Formula I-1 in which n is greater than 0 is greater than 0, and this proportion increases as the proportion of the aspartic acid increases.

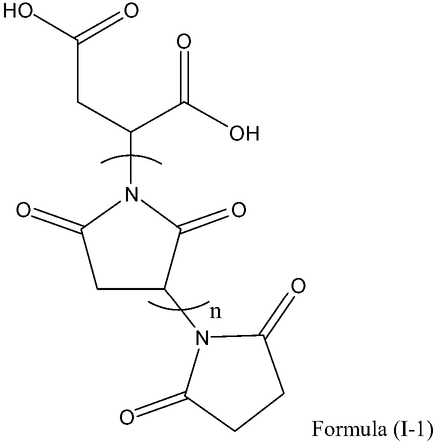

### Monomer Synthesis Example 3

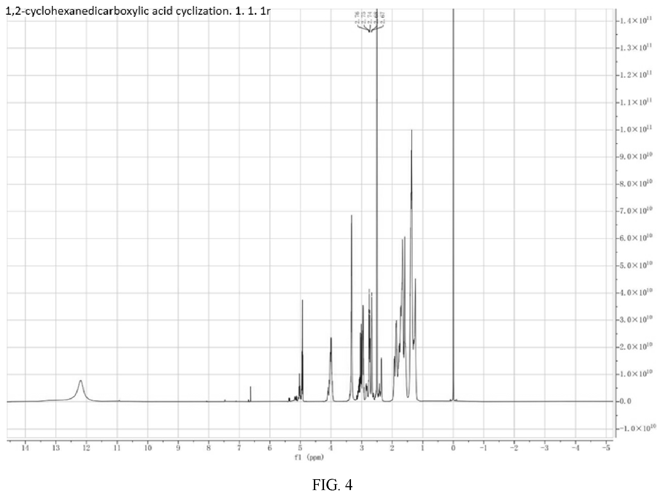

Aspartic acid and 1,2-cyclohexanedicarboxylic acid reacted in a reactor according to a molar ratio of 1:3, 200 ppm each of an antioxidant and a thermal stabilizer were added, and a resulting mixture was heated to melt under nitrogen protection so as to react. An NMR spectrum of a resulting product is shown in FIG. 4.

### Monomer Synthesis Example 4

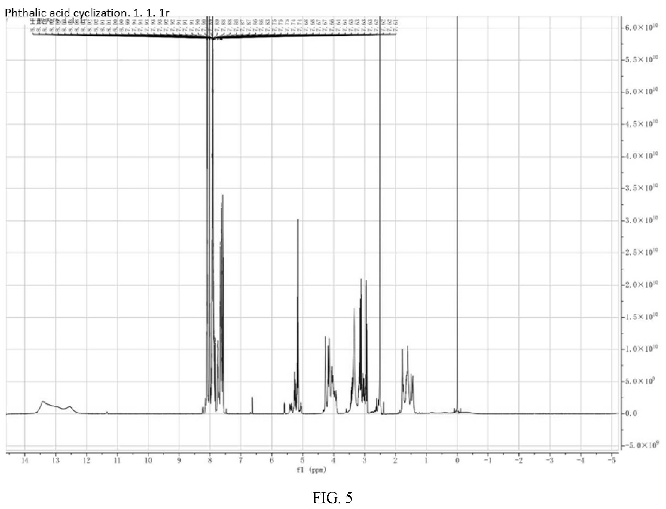

Aspartic acid and phthalic acid reacted in a reactor according to a molar ratio of 1:3, 200 ppm each of an antioxidant and a thermal stabilizer were added, and a resulting mixture was heated to melt under nitrogen protection so as to react. An NMR spectrum of a resulting product is shown in FIG. 5.

As can be seen from FIG. 4 and FIG. 5, hydrogen absorption peaks on a ring of cyclohexanedicarboxylic acid and phthalic acid are both shifted, and an amino peak of the aspartic acid disappears, indicating that compounds of Formula I-2 and Formula I-3 having side chains terminated with the cyclohexanedicarboxylic acid and the phthalic acid respectively are produced.

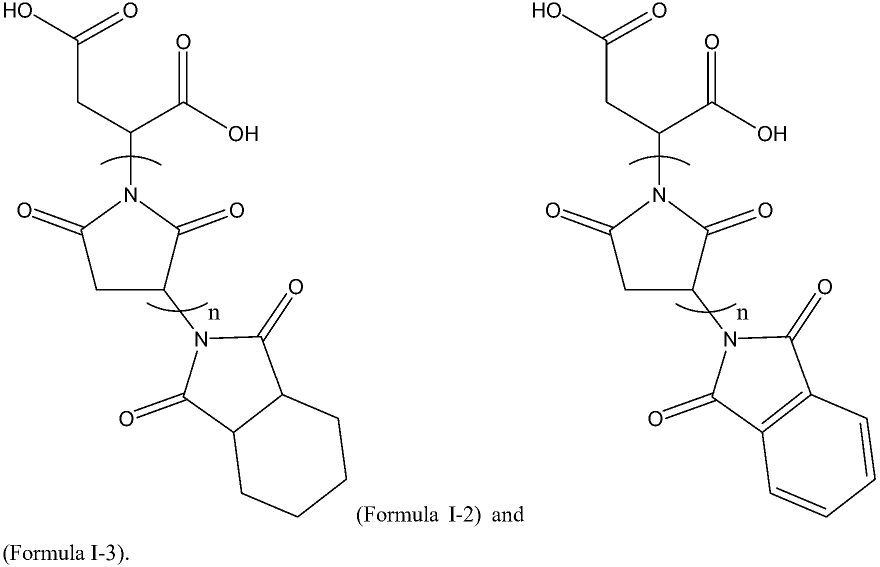

### Polymer Synthesis Example 1

First step: simultaneously with monomer synthesis, butanedioic acid and butanediol were added into another reactor with a molar ratio controlled at 1:1.2, and 200 ppm each of an antioxidant and a thermal stabilizer were added. A resulting mixture was heated to melt under nitrogen protection to carry out an esterification reaction.

Second step: materials from both reactors were blended and heated to 160°C, 200 ppm of anhydrous zinc acetate was added, butanediol was supplemented to control a ratio of diol : diacid = 1.2:1 of the system, and esterification was continued for 1 h to 2 h.

Third step: after the esterification was completed, 200 ppm of tetrabutyl titanate was added as a catalyst to carry out a polycondensation reaction, a vacuum degree inside the reactor was gradually reduced, the temperature was further raised to 250°C, and the reaction was maintained at a vacuum degree of 50 Pa or below for 4 to 6 h and then terminated to obtain a modified product. A structure thereof was:

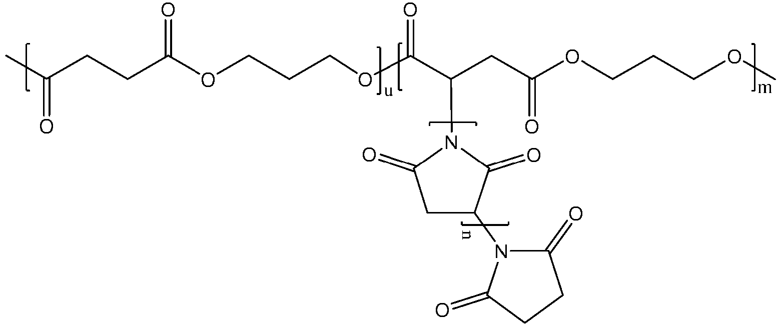

### Polymer Synthesis Example 2

First step: simultaneously with monomer synthesis, terephthalic acid and ethylene glycol were added into another reactor, 200 ppm each of an antioxidant, a thermal stabilizer and anhydrous zinc acetate were added, nitrogen was gradually charged to 150 Kpa in the stirring process, and the temperature was raised to 220°C to perform pressurized esterification.

Second step: after esterification was completed, materials from both reactors were mixed, and ethylene glycol was added to regulate and control a ratio of diol : diacid = 1.2:1. At 200°C, 200 ppm of antimony trioxide was added. When the amount of reaction water reached 99% of a theoretical value, the pressure was restored to atmospheric pressure, and a polycondensation reaction was carried out. A vacuum degree inside the reactor was slowly reduced, and the temperature was further raised to 250°C. The reaction was maintained at a vacuum degree of 50 Pa or below for 2 to 4 h and then terminated, and a resulting material was discharged to obtain modified PET resin. A structure was as follows:

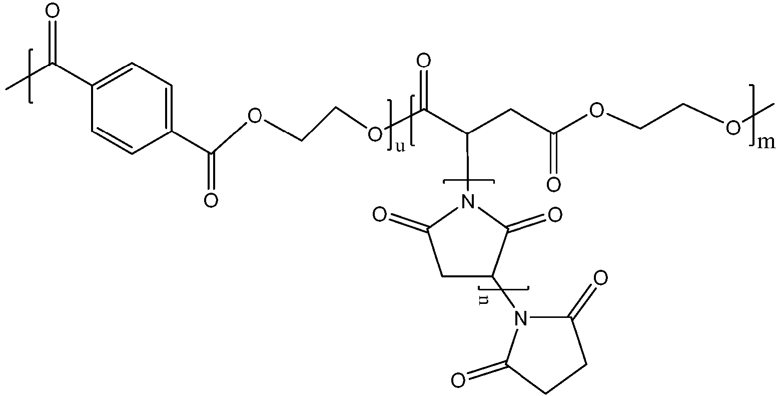

### Polymer Synthesis Example 3 (one-pot random copolymerization)

### First step: monomer synthesis was carried out.

Second step: after the monomer synthesis was completed, remaining butanedioic acid and butanediol were supplemented, and a proportion of an aspartic acid-modified segment was controlled at 10%. A ratio of diol : diacid of the system was controlled at 1.2:1. After 200 ppm of zinc acetate was added, an esterification reaction was carried out at 180°C for 2 to 4 h.

Third step: after the esterification was completed, 200 ppm of tetrabutyl titanate was added as a catalyst to carry out a polycondensation reaction, a vacuum degree inside the reactor was gradually reduced, the temperature was further raised to 250°C, and the reaction was maintained at a vacuum degree of 50 Pa or below for 4 to 6 h and then terminated.

### Effect Example

### Effect Example 1: Influence of different modifying unit ratios on properties of PBS polyester

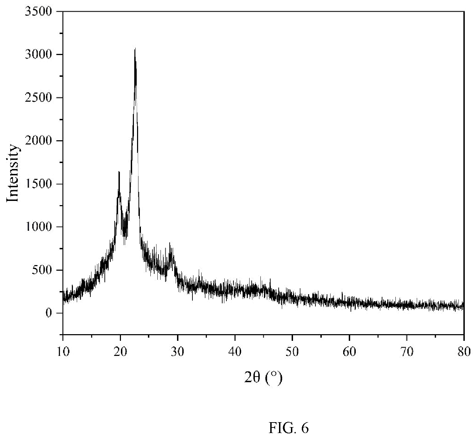

PBS polyesters with different modifying unit ratios (Example 1-1 to Example 1-7) were synthesized according to the method in Polymer Synthesis Example 1. The polymer in Example 1-8 was synthesized according to Polymer Synthesis Example 3. PBS was synthesized by a conventional method as a control. An XRD pattern of PBS modified with a 10% aspartic acid modifying unit is shown in FIG. 6: diffraction peaks at 19.7, 21.88 and 22.52 correspond to crystal planes 020, 021 and 110 of an α-crystalline form of PBS respectively. Characteristic peaks are distinct, indicating that a main crystalline component of the material is PBS. An FTIR spectrum is shown in FIG. 7, and no absorption peak is observed at 3050-3500 cm⁻¹ and 1660-1640 cm⁻¹, i.e., there is no absorption of primary amine groups or secondary amide groups, indicating that the aspartic acid in the polymer has been completely cyclized. Referring to Examples 1-1 to 1-7 in Table 1, when the content of the aspartic acid modifying unit is 5%, the heat resistance temperature of the material is 115.3°C (Example 1-1); and when the content of the aspartic acid modifying unit is 10%, the heat resistance of the material reaches an optimal value of 120.5°C (Example 1-2), and the tensile strength is also significantly improved. As the amount of modifying additives increases, the regularity of the material is disrupted to a greater extent, and the heat resistance decreases to some extent, but a high elongation at break (>300%) can be maintained while maintaining a relatively high tensile strength. The crystallization half-time is longer and can be regulated by the ratio, making it more suitable for the preparation of film materials. Moreover, since the amount of butanedioic acid used is reduced, the cost can be effectively lowered. When the content of the aspartic acid modifying unit is greater than 70%, the regularity of the material increases, and the heat resistance is increased back to 90°C or above. In addition, more imide rings can provide better hydrophilicity, which, while reducing costs, can expand applications in the field of hydrophilic polymer materials.

### Effect Example 2: Influence of precursor ratio (aspartic acid : butanedioic acid) of modifying monomer on properties of modified polymer

Monomers with different precursor ratios were synthesized with reference to Monomer Synthesis Example 1 or 2, and modified polymers were synthesized with reference to Polymer Synthesis Example 1. Referring to Table 2, the results show that when the ratio of aspartic acid : butanedioic acid is increased from 1:5 to 1:3, a melting point of the material is significantly elevated. When the ratio is further increased to 1:1, the heat resistance of the material decreases but is close to that of the comparative example of PBS. From the analysis of FIG. 2 and FIG. 3, it can be seen that when the proportion of the aspartic acid increases, more self-polymerized imide rings are formed, i.e., a moderate number of imide rings can provide the material with both good heat resistance and mechanical strength.

### Effect Example 3: Influence of modifying monomers with different precursors (cyclohexanedicarboxylic acid and phthalic acid) on properties of modified polymer

Monomers with different precursors were synthesized with reference to Monomer Synthesis Example 3 or 4, and modified polymers were synthesized with reference to Polymer Synthesis Example 1. Referring to Table 3, the results show that the tensile strength of the synthesized materials is significantly improved. At the same time, because the introduction of cyclohexanedicarboxylic acid and phthalic acid disrupts the regularity to some extent, a melting point is lower than that of a polymer modified with a monomer having a side chain terminated by butanedioic acid.

### Effect Example 4: Study on modification of different polyesters

Different modified polyesters were synthesized with reference to Polymer Synthesis Example 2. Referring to Table 4, the results show that after the introduction of modifying units, the elongation at break of different polyester materials is increased compared with the comparative example, and the toughness is effectively improved. Meanwhile, with respect to crystallization performance tests, the introduction of the modifying units can shorten the crystallization half-time, and the crystallization half-time of PBT increased from 54 s to 180 s.

**Table 1 Influence of content of aspartic acid modifying unit on properties of compounds**

| Polymer composition and property parameter | | Different aspartic acid modifying unit contents | | | | | | | | |
|---|---|---|---|---|---|---|---|---|---|---|
| | | 5% | 10% | 20% | 30% | 50% | 70% | 90% | (One-pot random copolymerization) | |
| | | Example 1-1 | Example 1-2 | Example 1-3 | Example 1-4 | Example 1-5 | Example 1-6 | Example 1-7 | Examples 1-8 | Comparative Example 1 |
| Modified segment | Cyclized diacid /anhydride | Butanedioic acid | Butanedioic acid | Butanedioic acid | Butanedioic acid | Butanedioic acid | Butanedioic acid | Butanedioic acid | Butanedioic acid | - |
| | n aspartic acid : n diacid /anhydride | 1:3 | 1:3 | 1:3 | 1:3 | 1:2 | 1:1 | 1:1 | 1:3 | |
| Main polyester segment | diacid | Butanedioic acid | Butanedioic acid | Butanedioic acid | Butanedioic acid | Butanedioic acid | Butanedioic acid | Butanedioic acid | Butanedioic acid | Butanedioic acid |
| | Diol | Butanediol | Butanediol | Butanediol | Butanediol | Butanediol | Butanediol | Butanediol | Butanediol | Butanediol |
| Segment molar ratio | n (imide ring segment) : n (ring structure polyester main chain) | 1:19 | 1:9 | 1:4 | 3:7 | 1:1 | 7:3 | 9:1 | 1:9 | |
| Alcohol-acid ratio in system during polycondensation | n-OH : n-COOH | 1.2:1 | 1.2:1 | 1.2:1 | 1.2:1 | 1.2:1 | 1.2:1 | 1.2:1 | 1.2:1 | 1.2:1 |
| Melt index (g/10 min) | | 5±2 | 5±2 | 5±2 | 5±2 | 30±2 | 50±2 | 100±2 | 10±2 | 5±2 |
| Material property | Tg/°C | -19.3 | -15.9 | -24.9 | -27.5 | -32.3 | None | None | -19.5 | -35.6 |
| | Tm/°C | 115.3 | 120.5 | 114.6 | 112.8 | 103.64 | None | None | 112.2 | 114.5 |
| | Tensile strength/MPa | 65.5 | 68.0 | 62.2 | 55.7 | 46.3 | 31.5 | 20.8 | 42.5 | 37.0 |
| | Elongation at break/% | 328.4 | 266.7 | 354.2.5 | 377.8 | 390.6 | 207.2 | 146.1 | 420.4 | 502.1 |

**Table 2 Influence of synthesis ratio of modifying monomer on property of modified polymer**

| Polymer composition and property parameter | | Synthesis ratio of modifying monomer and influence on polymerization | | | |
|---|---|---|---|---|---|
| | | Causing different values of n (the degree of aspartic acid self-polymerization) | | | |
| | | Example 2-1 | Example 2-2 | Example 2-3 | Example 2-4 |
| Modified segment | Cyclized diacid /anhydride | Butanedioic acid | Butanedioic acid | Butanedioic acid | Butanedioic acid |
| | n aspartic acid : n diacid /anhydride | 1:2 | 1:5 | 1:1 | 1:10 |
| Polyester main chain | diacid | Butanedioic acid | Butanedioic acid | Butanedioic acid | Butanedioic acid |
| | Diol | Butanediol | Butanediol | Butanediol | Butanediol |
| Segment molar ratio | n (imide ring segment) : n (ring structure polyester main chain) | 1:9 | 1:9 | 1:9 | 1:9 |
| Alcohol-acid ratio in system during polycondensation | n-OH : n-COOH | 1.2:1 | 1.2:1 | 1.2:1 | 1.2:1 |
| Melt index (g/10 min) | | 5±2 | 5±2 | 5±2 | 5±2 |
| Material property | Tg/°C | -16.7 | -18.6 | -19.4 | -23.6 |
| | Tm/°C | 119.9 | 115.6 | 113.9 | 111.3 |
| | Tensile strength/MPa | 57.5 | 55.2 | 55.4 | 45.7 |
| | Elongation at break/% | 350.1 | 390.4 | 331.2 | 398.7 |

**Table 3 Influence of modifying monomers with different precursors on property of modified polymer**

| Polymer composition and property parameter | | Cyclization of different precursors with aspartic acid | | |
|---|---|---|---|---|
| | | Example 3-1 | Example 3-2 | Example 3-3 |
| Modified segment | Cyclized diacid /anhydride | Butanedioic anhydride | 1,2-cyclohexanedicarboxy lic acid | Phthalic acid |
| | n aspartic acid : n diacid /anhydride | 1:3 | 1:3 | 1:3 |
| Polyester main chain | diacid | Butanedioic acid | Butanedioic acid | Butanedioic acid |
| | Diol | Butanediol | Butanediol | Butanediol |
| Segment molar ratio | n (imide ring segment) : n (ring structure polyester main chain) | 1:9 | 1:9 | 1:9 |
| Alcohol-acid ratio in system during polycondensation | n-OH : n-COOH | 1.2:1 | 1.2:1 | 1.2:1 |
| Melt index (g/10 min) | | 5±2 | 5±2 | 5±2 |
| Material property | Tg/°C | -18.9 | -20.2 | -19.3 |
| | Tm/°C | 119.6 | 111.2 | 112.2 |
| | Tensile strength/MPa | 57.3 | 54.4 | 54.2 |
| | Elongation at break/% | 329.3 | 302.3 | 293.5 |

**Table 4 Study on modification of different polyesters**

| Polymer composition and property parameter | | Modification of polyesters such as PET, PBT and PEF | | | | PET | PBT | PEF |
|---|---|---|---|---|---|---|---|---|
| | | Example 4-1 | Example 4-2 | Example 4-3 | Example 4-4 | Comparative Example 2 | Comparative Example 3 | Comparative Example 4 |
| Modified segment | Cyclized diacid /anhydride | Butanedioic acid | Butanedioic acid | Butanedioic acid | Butanedioic acid | | | |
| | n aspartic acid : n diacid /anhydride | 1:3 | 1:1 | 1:1 | 1:1 | | | |
| Polyester main chain | diacid | Terephthalic acid | Terephthalic acid | Terephthalic acid | 2,5-furandicarboxylicr acid | 1,4-benzenedicarboxylic acid | 1,4-benzenedicarboxylic acid | 2,5-furandicarboxylic acid |
| | Diol | Ethylene glycol | Ethylene glycol | Butane diol | Ethylene glycol | Ethylene glycol | Butanediol | Ethylene glycol |
| Segment molar ratio | n (imide ring segment) : n (ring structure polyester main chain) | 1:9 | 1:9 | 1:9 | 1:9 | | | |
| Alcohol-acid ratio in system during polycondensation | n-OH : n-COOH | 1.2:1 | 1.2:1 | 1.2:1 | 1.2:1 | 1.2:1 | 1.2:1 | 1.2:1 |
| Intrinsic viscosity (dL/g) | | 0.85 | 0.90 | 0.92 | 0.83 | 0.75 | 0.80 | 0.70 |
| Material property | Tg/°C | 74.0 | 79.2 | 51.2 | 84.6 | 70.4 | 55.6 | 81.8 |
| | Tm/°C | 217.6 | 220.8 | 238.9 | 210.3 | 245.2 | 233.5 | 215.0 |
| | Tensile strength/MPa | 60.3 | 63.4 | 58.3 | 82.6 | 58.7 | 52.1 | 80.1 |
| | Elongation at break/% | 46.5 | 27.9 | 270.2 | 29.3 | 7.3 | 268.5 | 8.1 |

## Claims

1. A diacid monomer containing an imide ring structure represented by Formula I, or an ester or prepolymer thereof, 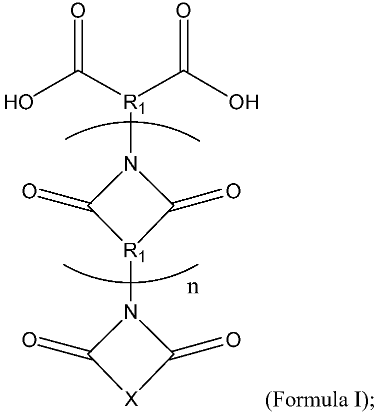
wherein R₁ is a derived group of aspartic acid, and X is a residue of a readily cyclizable diacid or -C=C-;
n is 0 or an integer greater than 0; and preferably, n is any integer from 0 to 7;
or, the monomer comprises one or more structures obtained after ring opening of the diacid monomer containing the imide ring structure represented by Formula I or the ester or prepolymer thereof, for example: 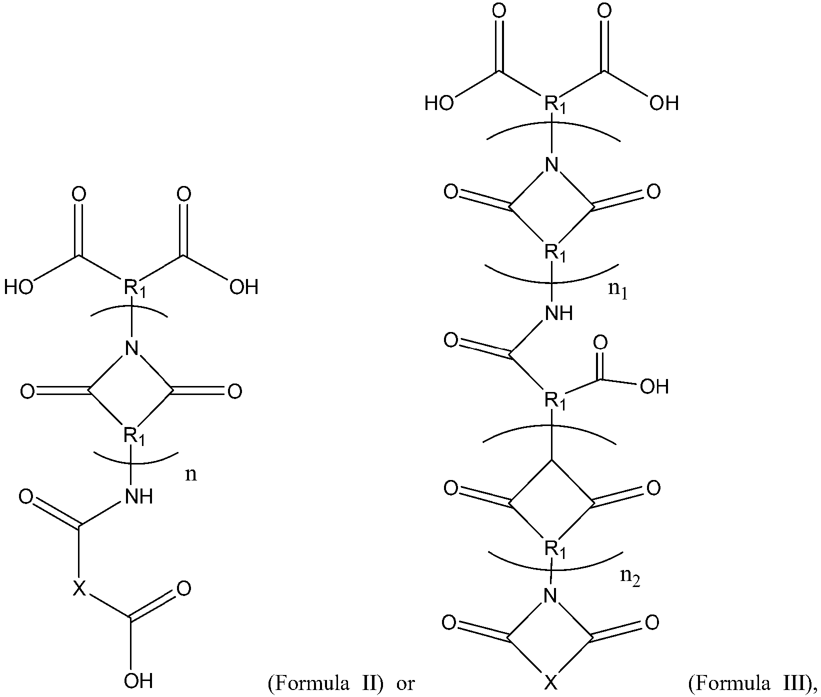 or a combination of ring opening manners of (Formula II) and (Formula III), wherein n₁ and n₂ are each a non-negative integer less than n, and preferably n₁ and n₂ are each any integer from 0 to 6;
an N value is independently selected from n, n₁ or n₂; and preferably the content of monomers with the N value greater than 0 is greater than 0;
X is preferably -C=C-; or X is preferably at least one of alkylene unsubstituted or substituted by a substituent selected from halogen, alkyl, aryl, arylalkyl or alkylaryl, alkylene unsubstituted or substituted by a substituent selected from halogen, alkyl, aryl, arylalkyl or alkylaryl and interrupted by one or more O atoms, alkenylene unsubstituted or substituted by a substituent selected from halogen, alkyl or nitro, cycloalkylene or heterocycloalkylene unsubstituted or substituted by a substituent selected from halogen, alkyl or nitro, cycloalkenylene unsubstituted or substituted by a substituent selected from halogen, alkyl or nitro, arylene unsubstituted or substituted by a substituent selected from halogen, alkyl or nitro, heteroarylene unsubstituted or substituted by a substituent selected from halogen, alkyl or nitro, or a bridged cyclic group unsubstituted or substituted by a substituent selected from halogen, alkyl or nitro;
preferably, the alkylene in definition of X is C1-C10 alkylene, preferably C1-C6 alkylene, more preferably C1-C5 alkylene, and most preferably methylene, ethylene, 1-methylethylene, 1,1-dimethylethylene, 1,2-dimethylethylene, 1,1,2,2-tetramethylethylene, 1-phenylethylene, 1-phenylmethylethylene, 1,1-diphenylethylene, 1,1-diphenylmethylethylene, 1,2-diphenylethylene, 1,2-diphenylmethylethylene, propylene, 1-methylpropylene, 2-methylpropylene, 1,1-dimethylpropylene, 1,2-dimethylpropylene, 2,2-dimethylpropylene, 1,3-dimethylpropylene, 1-phenylpropylene, 2-phenylpropylene, 1,2-diphenylpropylene, 2,2-diphenylpropylene, or 1,3-diphenylpropylene; the alkenylene in the definition of X is C2-C10 alkenylene, preferably C2-C6 alkenylene, more preferably C2-C3 alkenylene, and most preferably vinylene, 1-methylvinylene, 1,2-dimethylvinylene, or propenylene; the alkylene unsubstituted or substituted by the substituent selected from halogen, alkyl, aryl, arylalkyl or alkylaryl and interrupted by one or more O atoms in the definition of X is interrupted by 1 to 3 oxygen atoms, such as 2-oxa-1,3-propylene; the cycloalkylene in the definition of X is C3-C8 cycloalkylene, preferably C4-C6 cycloalkylene, and most preferably cyclobutylene or cyclohexylene; the cycloalkenylene in the definition of X is C4-C8 cycloalkenylene, preferably C4-C6 cycloalkenylene, and most preferably 3-cyclohexene-1,6-diyl; the arylene in the definition of X is C6-C10 arylene, preferably phenylene, such as 1,2-phenylene; and bridged cycloalkylene in the definition of X is norbornenylene;
most preferably, X is -C=C-, a butanedioic acid residue (ethylene), a 1,2-cyclohexanedicarboxylic acid residue (1,2-cyclohexylene) or a phthalic acid residue (1,2-phenylene); and
preferably, the aspartic acid in the monomer is of biological origin; and preferably, the aspartic acid has a ¹⁴C/¹²C ratio greater than 0.

2. A monomer composition, comprising the diacid monomer containing the imide ring structure represented by Formula I , or further comprising diacid monomer represented by Formula II and/or Formula III, or the ester or prepolymer thereof according to claim 1, and comprising two or more monomers with different N values, wherein the N value is independently selected from n, n₁ or n₂, and the content of monomers with the N value greater than 0 is greater than 0;
preferably, the monomer composition is the diacid monomer containing the imide ring structure represented by Formula I or represented by a mixture of Formula I and Formula II or the ester or prepolymer of the monomer, more preferably a mixture of monomers comprising at least one N value of n being 0 or n being 1 to 7, or more preferably a mixture of monomers comprising at least two N values of n being 1 to 7;
or, the monomer composition is the diacid monomer containing the imide ring structure represented by a mixture of Formula I and Formula III or a mixture of Formula I, Formula II and Formula III or the ester or prepolymer of the monomer, more preferably a mixture of monomers comprising at least one N value of n being 0 to 7 and monomers comprising at least one N value of n₁ and n₂ being 0 to 6, and most preferably a monomer containing at least one N value of n, n₁ and n₂ being greater than 0;
preferably, the monomer or the monomer composition comprises at least two, three, four, five or six monomers with an N value of 0, 1, 2, 3, 4 or 5;
preferably, the monomer or the monomer composition comprises monomers with an N value of 0, 1 and 2; and preferably, the content of the monomers with the N value of 0 is the maximum;
preferably, the monomer or the monomer composition comprises monomers with an N value of 1, 2, 3, 4 and 5; and preferably, the content of the monomers with the N value of 2 is the maximum; and
preferably, the monomer or the monomer composition comprises monomers with an N value of 2, 3, 4 and 5; and preferably, the content of the monomers with the N value of 3 is the maximum.

3. A copolymer, comprising a polymer backbone structural unit and a first modified structural unit, wherein the first modified structural unit contains a structure shown in Formula VI: 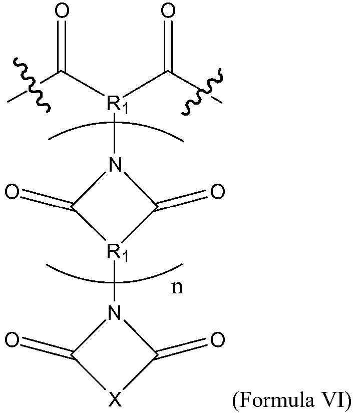
wherein R₁ is a derived group of aspartic acid, and X is a residue of a readily cyclizable diacid or -C=C-;
preferably, the first modified structural unit is a polyester structure shown in (Formula VI-1): 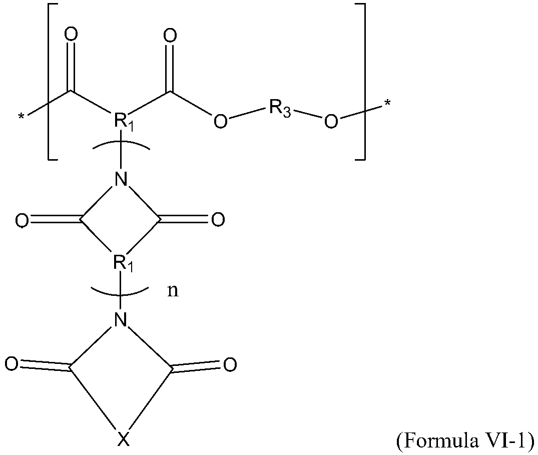
R₃ is a residue of a diol for polymer synthesis, and preferably is selected from C1-C12 alkylene unsubstituted or substituted by a substituent selected from halogen, alkyl, aryl, arylalkyl or alkylaryl, cycloalkylene, or heterocyclylene comprising at least one N, O or S atom; and preferably, R₃ is selected from at least one of C2-C8 alkylene unsubstituted or substituted by a substituent selected from halogen, alkyl, aryl, arylalkyl or alkylaryl, C4-C8 cycloalkylene or C5-C8 heterocyclylene comprising an oxygen atom, and is further preferably C2-C6 alkylene;
preferably, the first modified structural unit further contains a structure shown in Formula VII and/or Formula VIII: 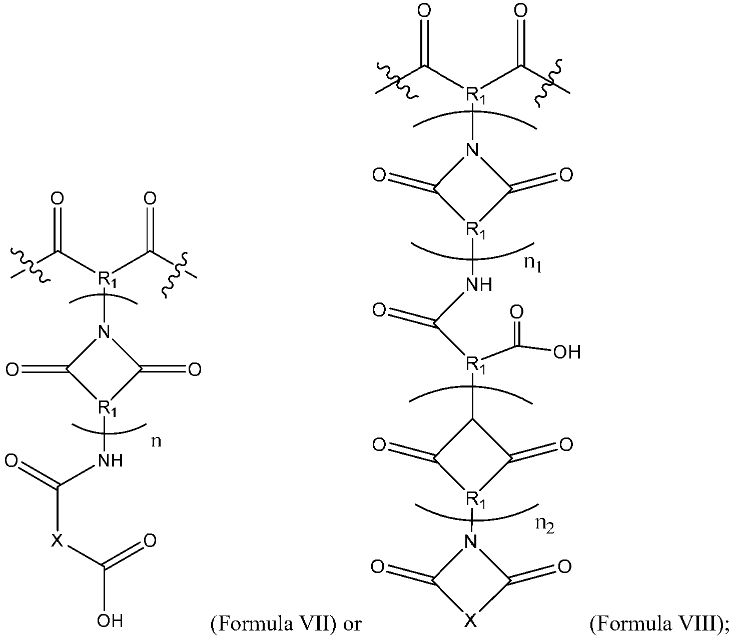
n is 0 or an integer greater than 0, and preferably n is any integer from 0 to 7; n₁ and n₂ are each a non-negative integer less than n, and preferably n₁ and n₂ are each any integer from 0 to 6; and preferably, an N value is independently selected from n, n₁ or n₂, and the content of monomers with the N value greater than 0 is greater than 0;
preferably, the first modified structural unit further contains a polyester structure shown in (Formula VII-1) and/or (Formula VIII-1): 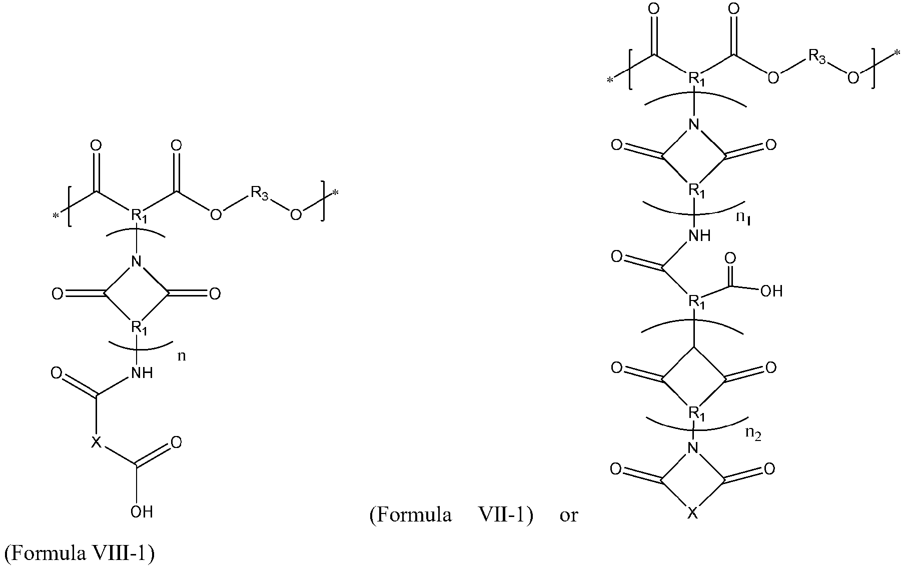
and preferably, the first modified structural unit forms a branched and/or cross-linked structure through double bonds and/or a carboxyl group on a side chain.

4. The copolymer according to claim 3, wherein the polymer backbone structural unit is polyester;
preferably, the backbone structural unit is polyester such as one or more of aliphatic polyester, aliphatic-aromatic polyester, and aromatic polyester; further preferably, the backbone structural unit contains two or more different structural units; and
preferably, the backbone structural unit is a structural unit of one or more polyesters of PBS, PES, PBA, PET, PBT, PTT, PBAT, PBST, PEAT or PEST.

5. The copolymer according to claim 3 or 4, wherein the first modified structural unit accounts for 0.5-99.5mol% of total polymer structural units, preferably 1-90 mol%, and further preferably 5-30 mol%.

6. The copolymer according to any one of claims 3 to 5, further comprising a second modified structural unit.

7. A polymer alloy, comprising the copolymer according to any one of claims 3 to 6.

8. A polymer composition or molded article, comprising the copolymer according to any one of claims 3 to 6 or the polymer alloy according to claim 7.

9. Use of the copolymer according to any one of claims 3 to 6, the polymer alloy according to claim 7 or the composition or molded article according to claim 8 in food containers, food packaging films, disposable tableware such as spoons or straws, daily necessities, cosmetics, transparent boxes for household appliances and the like, packaging containers such as transparent windows of paper boxes, stationery such as transparent folders and card holders, industrial films or agricultural films, and chemical fibers for clothing or industrial use.

10. Use of the monomer or the ester or prepolymer thereof according to claim 1, or the monomer composition according to claim 2, or a precursor composition of the monomer or the monomer composition for polymer synthesis, wherein the precursor composition comprises aspartic acid and a readily cyclizable diacid;
preferably, the monomer or the monomer composition comprises monomers with an N value of 0, 1 and 2; and preferably, the monomers with the N value of 0 have the highest content;
preferably, the monomer or the monomer composition comprises monomers with an N value of 1, 2, 3, 4 and 5; and preferably, the monomers with the N value of 2 have the highest content; and
preferably, the monomer or the monomer composition comprises monomers with an N value of 2, 3, 4 and 5; and preferably, the monomers with the N value of 3 have the highest content.

11. A method for synthesizing the monomer according to claim 1 or the monomer composition according to claim 2, comprising a step of reacting aspartic acid with a readily cyclizable diacid or an anhydride thereof, wherein preferably, a molar ratio of the aspartic acid to the readily cyclizable diacid is 1:50-50:1, preferably 1:10-10:1, and further preferably 1:3-1:1.

12. A method for synthesizing the copolymer according to any one of claims 3 to 6, comprising:
1) providing the monomer according to claim 1 or the monomer composition according to claim 2;
2) providing a polyester prepolymer of a diacid and a diol; and
3) subjecting the monomer or the monomer composition in Step 1), the prepolymer in Step 2) and optionally a newly added diacid and/or diol to a polycondensation reaction, wherein
preferably, the monomer according to claim 1 or the monomer composition according to claim 2 is provided using the method according to claim 11.
